# EUROPÄISCHE PATENTSCHRIFT

(11) **EP 0 977 528 B1**
(45) Veröffentlichungstag und Bekanntmachung des Hinweises auf die Patenterteilung: **02.05.2002**
(21) Anmeldenummer: 99908828.9
(22) Anmeldetag: 27.01.1999
(51) Int. Cl.: A61F 2/44, A61F 2/30, A61F 2/46

(54) **PLATZHALTER MIT EINSTELLBARER AXIALER LÄNGE**
SPACER WITH ADJUSTABLE AXIAL LENGTH
ESPACEUR A LONGUEUR AXIALE REGLABLE

(30) Priorität: 06.02.1998 DE 19804765
(43) Veröffentlichungstag der Anmeldung: 09.02.2000
(73) Patentinhaber: BIEDERMANN MOTECH GmbH, 78054 Villingen-Schwenningen (DE)
(72) Erfinder: BIEDERMANN, Lutz, D-78048 Villingen-Schwenningen (DE); HARMS, Jürgen, D-76337 Waldbronn (DE)
(74) Vertreter: Prüfer, Lutz H., Dipl.-Phys.
(86) Internationale Anmeldenummer: EP9900526
(87) Internationale Veröffentlichungsnummer: WO9939665

(56) Entgegenhaltungen:
- EP-A- 0 290 767
- EP-A- 0 490 159
- WO-A-92/01428
- DE-A- 19 519 101
- DE-A- 19 622 827
- DE-U- 29 616 778
- US-A- 4 599 086
- US-A- 5 569 263

## Beschreibung

Die Erfindung betrifft einen Platzhalter zum Einsetzen insbesondere zwischen zwei Wirbelkörper mit einer veränderbaren axialen Länge, mit einem hülsenartigen ersten Teil und einem in diesem geführten und in axialer Richtung relativ zum ersten Teil verschiebbaren zweiten Teil zum Einstellen einer Gesamtlänge.

Ein solcher Platzhalter ist aus der DE 196 22 827 A1 bekannt. Das in dem ersten Teil geführte zweite Teil weist auf seiner Oberfläche ein Gewinde auf, auf welches ein Anschlagring geschraubt ist. Das erste Teil wird auf das zweite Teil aufgesteckt, und die Teile werden bis zu dem durch den aufgeschraubten Ring gebildeten Anschlag ineinander geschoben. Nach dem Einsetzen zwischen die beiden Wirbelkörper erfolgt die endgültige Längenanpassung dadurch, daß der Ring zum Verlängern des Platzhalters in Richtung zu der ersten Hülse hin geschraubt wird. In der Endstellung werden die beiden Teile mittels einer Fixierschraube relativ zueinander verschraubt. Der Platzhalter weist an seinen beiden freien Enden Stirnplatten auf, an deren Stirnfläche zum Eindringen in den angrenzenden Wirbel Schneiden vorgesehen sind. Durch das Erfordernis des Drehens des Anschlagringes um die Längsachse wird auch auf die beiden Teile ein gewisses Drehmoment ausgeübt, was zur Folge hat, daß bei dem Einsetzen des Platzhalters die Gefahr besteht, daß die angrenzenden Wirbelkörper mit ihren Schneiden an den freien Enden die angrenzenden Wirbelkörper durch das ersten Teil und einen in diesem geführten und in axialer Richtung relativ zum ersten Teil verschiebbaren zweiten Teil zum Einstellen einer Gesamtlänge. Beide Teile weisen Öffnungen auf. Die Öffnungen im inneren Teil weisen Gewinde auf, so daß nach Einstellen der gewünschten Länge bei Abgleich von äußeren und inneren Öffnungen eine Schraube durch die äußere Öffnung so eingesetzt werden kann, daß die Relativstellung der beiden Teile zueinander fixiert wird.

Aus der US 5,569,263 ist eine Einrichtung für Hüftgelenkimplantate bekannt, bei der ein kugelförmiges Element eine zylindrische Bohrung aufweist, in die ein entsprechendes Zylinderteil in einer gewünschten Länge eingesetzt und arretiert werden soll. Das zylindrische Element weist mehrere ringförmige Nuten auf, die in axialer Richtung gesehen in einem Abstand zueinander angeordnet sind. In einer Richtung senkrecht zu der Achse der Bohrung ist eine weitere Bohrung vorgesehen, in der eine Kugel eingeführt ist, die von einer Druckfeder beaufschlagt ist, welche wiederum von einer von außen zu betätigenden Abschlußschraube in vorspannung versetzt ist. Die Tiefe der Nuten ist gleich dem Radius der Kugel, die Breite der Nuten im wesentlichen gleich dem Durchmesser der Kugel. Auf diese Weise wird erreicht, daß bei Eingreifen der Kugel in die Nut eine weitere Relativbewegung zwischen Zylinder und Kugel verhindert wird.

Aufgabe der Erfindung ist es, die Einstellung der gewünschten Länge des Platzhalters zu vereinfachen.

Diese Aufgabe wird nach einer ersten Lösung durch den in Patentanspruch 1 gekennzeichneten Platzhalter gelöst. Nach einer zweiten Lösung wird die Aufgabe durch den in Patentanspruch 12 gekennzeichneten Platzhalter gelöst.

Weitere Merkmale und Zweckmäßigkeiten der Erfindung ergeben sich aus der Beschreibung eines Ausführungsbeispieles anhand der Figuren. Von den Figuren zeigen:
- Fig. 1: eine Seitenansicht eines Teiles des Platzhalters;
- Fig. 2: eine Draufsicht auf das in Fig. 1 gezeigte Teil von oben;
- Fig. 3: eine Seitenansicht des anderen Teiles des Platzhalters;
- Fig. 4: eine Ansicht des in Figur 3 gezeigten Teiles von unten;
- Fig. 5: eine Seitenansicht der ineinandergeschobenen beiden Teile;
- Fig. 6: einen Schnitt entlang der Linie A - A in Fig. 5 mit eingesetztem Werkzeug;
- Fig. 7: eine Seitenansicht einer Fixierschraube;
- Fig. 8: eine Draufsicht auf die Fixierschraube;
- Fig. 9: eine Seitenansicht des Platzhalters mit winkelmäßig getrimmten freien Enden;
- Fig. 10: den zwischen zwei Wirbelkörper eingesetzten Platzhalter mit Werkzeug;
- Fig. 11: den auf die richtige Länge getrimmten Platzhalter mit Fixierschraube;
- Fig. 12: eine vergrößerte Darstellung der eingesetzten Fixierschraube;
- Fig. 13: den Platzhalter mit aufgesetzten Endplatten; und
- Fig. 14: eine Draufsicht auf eine der Endplatten.

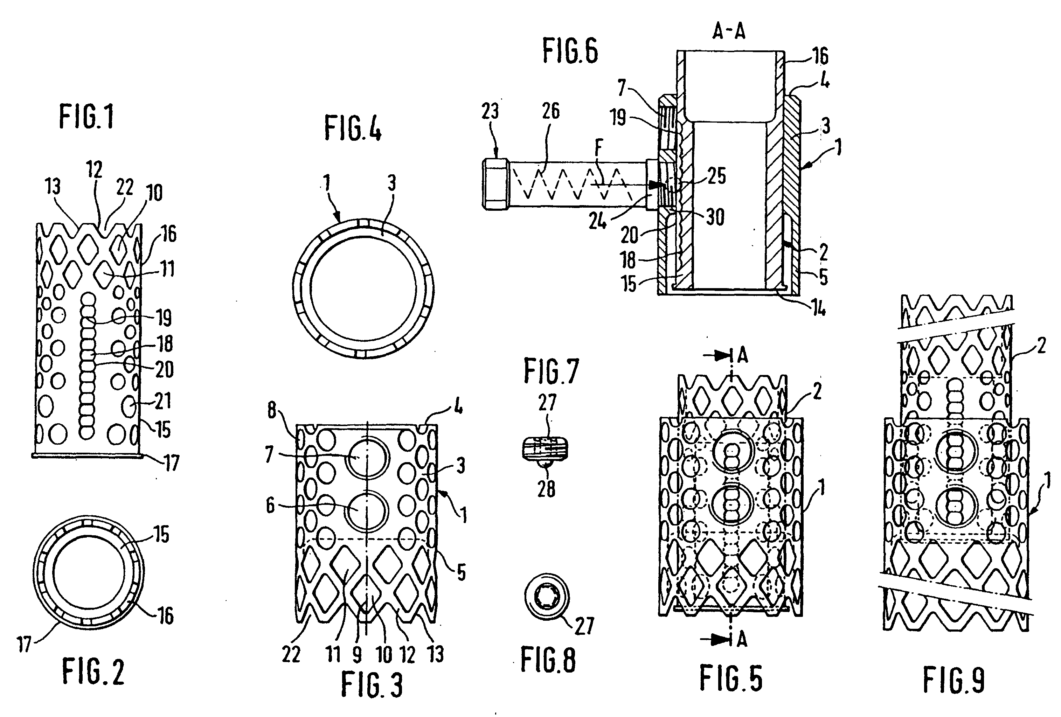

Wie am besten aus den Figuren 5 und 6 ersichtlich ist, weist der Platzhalter eine ein erstes Teil bildende äußere Hülse 1 und ein ein zweites Teil bildende innere Hülse 2 auf.

Wie am besten aus den Figuren 3, 4 und 6 ersichtlich ist, weist die äußere Hülse 1 einen ersten Mantelabschnitt 3 auf, der sich von dem ersten freien Ende 4 bis über die Hälfte der Hülse zu ihrem anderen Ende hin erstreckt. Dieser Mantelabschnitt weist einen ersten Innendurchmesser auf. In dem daran anschließenden zweiten Mantelabschnitt 5 ist der Mantel auf der Innenseite dünner ausgearbeitet und weist einen zweiten Innendurchmesser auf, der größer ist als der erste Innendurchmesser. Der erste Mantelabschnitt weist, wie es am besten aus Fig. 3 ersichtlich ist, zwei in axialer Richtung der Hülse übereinanderliegende Gewindebohrungen 6 und 7 auf. Ferner sind im ersten Mantelabschnitt eine Mehrzahl über den gesamten Mantelabschnitt verteilter Ausnehmungen 8 in Form von sich durch den Mantel erstreckenden Bohrungen vorgesehen. Der zweite Mantelabschnitt 5 weist in der aus Fig. 3 ersichtlichen Weise sich mit ihrer Längsdiagonale parallel zur Mantelachse 9 erstreckende rautenförmige Ausnehmungen 10, 11 auf. An den Rand angrenzend erstreckt sich in Umfangrichtung eine erste Gruppe von dieser rautenförmigen Ausnehmungen 10, und daran anschließend zum ersten freien Ende 4 hin gerichtet eine zweite Gruppe 11 der rautenförmigen Ausnehmung, wobei die zweite Gruppe zur ersten Gruppe um eine halbe Rautenhöhe in axialer Richtung versetzt sind. Dadurch wird ein Netz von sich unter einem spitzen Winkel schneidenden Bandstreifen 12, 13 gebildet, die unter jeweils gleichgroßen Winkeln gegen die Längsdiagonale der Rauten geneigt sind. Die Größe der Rauten und der diese begrenzenden Bandstreifen ist so gewählt, daß die Anzahl von Rauten in Umfangsrichtung stets ganzzahlig ist. Der so gebildete Rand weist damit von den Bandstreifen gebildete Zacken und dazwischen Vertiefungen 22 auf.

Das zweite Teil weist, wie es am besten aus den Figuren 1, 2 und 6 ersichtlich ist, einen an sein erstes freies Ende 14 anschließenden ersten Mantelabschnitt 15 mit einem ersten Außendurchmesser auf. Dieser Mantelabschnitt erstreckt sich in dem gezeigten Ausführungsbeispiel über etwas mehr als zwei Drittel der axialen Länge. Daran schließt sich ein bis zum zweiten freien Ende sich erstreckender zweiter Mantelabschnitt 16 mit dem gleichen Außendurchmesser an. Der Außendurchmesser ist so gewählt, daß die innere Hülse in der am besten aus Fig. 6 ersichtlichen Weise gleitend in dem ersten Mantelabschnitt 3 der äußeren Hülse geführt wird. An dem ersten freien Ende 14 weist die innere Hülse einen Rand 17 auf, dessen Durchmesser größer ist als der Durchmesser des ersten Mantelabschnittes und größer als der erste Innendurchmesser der äußeren Hülse und kleiner ist als der Innendurchmesser des zweiten Mantelabschnittes 5 der äußeren Hülse.

Der erste Mantelabschnitt 15 weist in einer Richtung parallel zur Mantelachse 9 eine Mehrzahl von unmittelbar aneinandergrenzenden kugelsegmentförmigen Vertiefungen 18 auf, deren Tiefe kleiner als ihr Radius ist. Wie am besten aus Fig. 1 ersichtlich ist, ist der Abstand jeweils zweier benachbarter Vertiefungen kleiner als der Durchmesser des die Vertiefung begrenzenden Randes der kugelsegmentförmigen Vertiefungen. Dadurch wird erreicht, daß die Grenze 19 zwischen zwei benachbarten Vertiefungen niedriger ist als der eigentliche Rand 20 der Vertiefungen selbst. Der Bereich der Vertiefungen 18 erstreckt sich über nahezu die gesamte Länge der ersten Mantelabschnittes 15. In diesem ersten Mantelabschnitt sind analog zu den Ausnehmungen 8 ebenfalls sich über die gesamte Fläche des Mantelabschnittes erstreckende als durch den Mantel hindurchgehende Bohrungen gebildete Ausnehmungen 21 angeordnet. Der zweite Mantelabschnitt 16 ist in analoger Weise zum zweiten Mantelabschnitt 5 der ersten Hülse ausgebildet und weist in gleicher Weise angeordnete rautenförmige Ausnehmungen 10, 11 auf, mit den. Bändern 12, 13 dazwischen. Die Bänder sind am freien Ende der Hülse schräg zueinander verlaufend ausgebildet und schließen jeweils Vertiefungen 22 mit dazwischen hervorstehenden relativ scharfen Kanten ein.

Zur Bildung des eigentlichen Platzhalters wird die innere Hülse 2 in der am besten aus den Figuren 5 und 6 ersichtlichen Weise von dem zweiten Mantelabschnitt 5 her in die äußere Hülse 1 eingeschoben. Dabei ist die maximale Einschubtiefe durch den Anschlag des Randes 17 an der zwischen den beiden Mantelabschnitten 3 und 5 gebildeten inneren Schulter begrenzt.

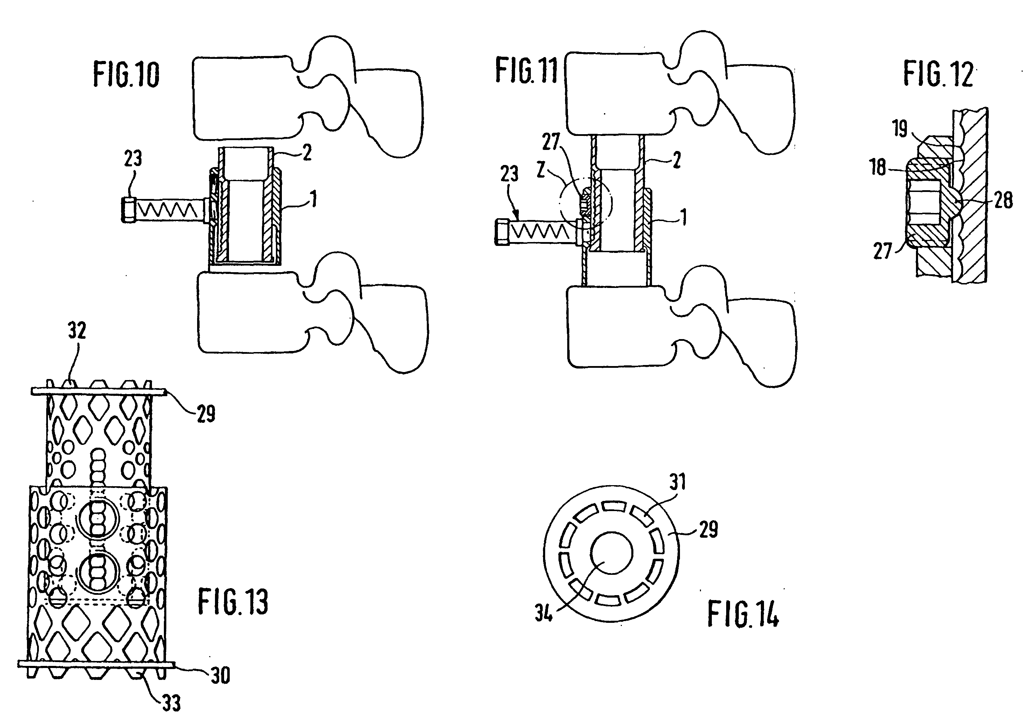

Damit der Platzhalter am besten aus den Figuren 10 und 11 ersichtlichen Weise zunächst zwischen die beiden angrenzenden Wirbelkörper eingesetzt und dann auf die gewünschte Länge expandiert und fixiert werden kann, ist ein Werkzeug 23 vorgesehen. Dieses weist an seinem einen Ende ein Außengewinde 30 auf, welches zu dem Innengewinde der beiden Gewindebohrungen 6, 7 paßt. Anschließend an das Außengewinde ist eine Schulter 24 vorgesehen, die beim Einschrauben des Werkzeuges in die Gewindebohrungen 6 oder 7 einen Anschlag bildet. Auf der an dem Außengewinde angrenzenden Stirnfläche ist eine Kugel 25 gehalten, die durch eine schematisch angedeutete innere Feder in Richtung des Pfeiles F die Kugel nach außen hin federnd vorspannt. Der Durchmesser der Kugel ist gleich und vorzugsweise etwas kleiner als der Durchmesser der kugelförmigen Vertiefungen 18, so daß die Kugel genau in die kugelsegementförmigen Abschnitte hineinpaßt. In diesem Zustand des Platzhalters können die beiden Hülsen relativ zueinander verschoben werden und, wie am besten aus den Figuren 5 und 9 ersichtlich ist, bis zu einer maximalen Länge vergrößert werden. Die federvorgespannte Kugel 25 wirkt mit den kugelsegmentförmigen Vertiefungen 18 nach Art einer Ratsche derart zusammen, daß die Vergrößerung jeweils um den Abstand zweier Vertiefungen oder eines mehrfachen davon verändert werden kann. Das Auseinanderziehen der beiden Hülsen erfolgt während der Operation durch Anwendung eines geeigneten Spreizwerkzeuges.

Es sind die in Fig. 7 und 8 gezeigten Fixierschrauben 27 vorgesehen, deren Außengewinde so gewählt ist, daß es mit den Gewindebohrungen 6, 7 zusammenwirkt. Auf einer Stirnseite der Fixierschraube ist, wie am besten aus den Figuren 7 und 12 ersichtlich ist, in der Mitte ein Kugelsegment 28 vorgesehen. Die Abmessungen des Kugelsegmentes 28 entsprechen dem ohne Gegenkraft aus der Stirnfläche des Werkzeuges 23 hervorstehenden Kugelsegment. Auf der gegenüberliegenden Stirnfläche ist eine Sechskantbohrung zum Eingreifen eines Schraubendrehers vorgesehen.

Ist im Betrieb der gemäß Figur 10 eingebrachte Platzhalter auf die in Fig. 11 gezeigte optimale Länge eingestellt und unter Verwendung des Werkzeuges 23 gehalten, dann wird zur endgültigen Fixierung in die zweite Gewindebohrung eine Fixierschraube 27 der in Fig. 12 gezeigten Weise fest eingeschraubt. Damit werden die beiden Hülsen relativ zueinander endgültig fixiert. Nach dem Herausdrehen des Werkzeuges 23 wird auch in die zweite Gewindebohrung eine entsprechende Schraube 27 zum zusätzlichen Fixieren eingebracht.

Wie aus Fig. 9 ersichtlich ist, bietet das Vorsehen der Abschnitte 5 und 16 mit den rautenförmigen Ausnehmungen die Möglichkeit, die winkelmäßige Neigung der an den Wirbelkörpern angreifenden Flächen mit geeignetem Schneidwerkzeug einzustellen und dabei am Rand Vertiefungen und Zacken zum Eingreifen in den angrenzenden Wirbelkörpern zu bilden.

Wie aus den Figuren 13 und 14 ersichtlich ist, sind Endplatten 29 und 30 vorgesehen, die in bekannter Weise Ausnehmungen 31 zum Aufsetzen auf die hervorstehenden Zacken 32, 33 sowie Ausnehmungen 34 zum Ermöglichen eines besseren Einwachsens aufweisen.

In dem oben beschriebenen Ausführungsbeispiel sind die beiden Hülsen zylinderförmig ausgebildet. Sie können aber auch einen anderen Querschnitt aufweisen, wobei der Querschnitt durch die zu verbindenden Teile bestimmt wird.

## Patentansprüche

1. Platzhalter zum Einsetzen zwischen zwei Wirbelkörper, mit einer veränderbaren axialen Länge, mit
einem hülsenartigen ersten Teil (1) und einem in diesem geführten und in axialer Richtung relativ zum ersten Teil verschiebbaren zweiten Teil (2) zum Einstellen einer Gesamtlänge,
**dadurch gekennzeichnet, daß** das zweite Teil (2) auf seiner dem ersten Teil (1) zugewandten Außenwand einen sich in axialer Richtung erstreckenden Abschnitt mit eine Rasterung (18) mit einer Mehrzahl von in axialer Richtung benachbart zueinander angeordneten und aneinander grenzenden Vertiefungen (18) und das erste Teil (1) ein mit der Rasterung (18) zum Verschieben auf eine gewünschte Gesamtlänge zusammenwirkendes Eingriffsteil (23) aufweist, wobei das Eingriffsteil in die Wandung des ersten Teiles (1) einsetzbar ist und ein federvorgespanntes Ratschenteil aufweist.

2. Platzhalter nach Anspruch 1, **dadurch gekennzeichnet, daß** ein mit der Rasterung (18, 19) zusammenwirkendes Arretierteil (27) zum Arretieren der beiden Teile in der gewünschten Gesamtlänge vorgesehen ist.

3. Platzhalter nach Anspruch 2, **dadurch gekennzeichnet, daß** das Ratschenteil als federvorgespannte Kugel (25) ausgebildet ist.

4. Platzhalter nach Anspruch 3, **dadurch gekennzeichnet, daß** die Vertiefungen hohlkugelsegmentförmig ausgebildet sind.

5. Platzhalter nach Anspruch 4, **dadurch gekennzeichnet, daß** der Abstand der Mittelpunkte zweier benachbarter Vertiefungen kleiner als der Kugeldurchmesser ist und vorzugsweise kleiner als der Durchmesser des Randes (20) der Vertiefung ist.

6. Platzhalter nach einem der Ansprüche 2 bis 5,
**dadurch gekennzeichnet, daß** das Arretierteil als eine Schraube (25) ausgebildet ist, die auf ihrer der Rasterung zugewandten Stirnfläche ein Kugelsegment (28) aufweist, welches so bemessen ist, daß es gerade in die Vertiefung (18) hineinpaßt.

7. Platzhalter nach einem der Ansprüche 2 bis 6,
**dadurch gekennzeichnet, daß** das erste Teil (1) eine erste Gewindebohrung (6) zum Aufnehmen des Eingriffsteiles (23) und eine in Richtung der Mittenachse (9) gesehen oberhalb oder unterhalb angeordnete zweite Gewindebohrung (7) zum Aufnehmen des Arretierteiles (25) aufweisen.

8. Platzhalter nach einem der Ansprüche 1 bis 7,
**dadurch gekennzeichnet, daß** die freien Enden der beiden Platzhalterteile jeweils zackenförmig ausgebildet sind.

9. Platzhalter nach einem der Ansprüche 1 bis 8,
**dadurch gekennzeichnet, daß** die Wandungen der beiden Platzhalterteile (1, 2) eine Mehrzahl von Ausnehmungen (8, 21) aufweisen, von denen in jeder Rasterstellung in Umfangsrichtung verteilt eine Mehrzahl wenigstens teilweise deckungsgleich ist.

10. Platzhalter nach einem der Ansprüche 1 bis 9,
**dadurch gekennzeichnet, daß** der Endabschnitt (5, 10) wenigstens eines der beiden Platzhalterteile (1, 2) rautenförmige Ausnehmungen (10, 11) aufweist und sich eine Diagonale der Rauten sich jeweils parallel zur Längsachse (9) des Platzhalters erstreckt.

11. Platzhalter nach einem der Ansprüche 1 bis 10, **dadurch gekennzeichnet, daß** ein Anschlag (17) zum Begrenzen der Relativverschiebung auf eine maximale Gesamtlänge vorgesehen ist.

## Claims

1. Spacer for insertion between two vertebral bodies and having a changeable axial length, having a sleeve-like first part (1) and a second part (2), which is guided in said first part and can be displaced axially relative to the first part, for setting an overall length, **characterized in that** on its outer wall, which is directed towards the first part (1), the second part (2) has an axially extending section with a latching arrangement (18) with a plurality of adjoining depressions (18) which are adjacent to one another in the axial direction, and the first part (1) has an engagement part (23) which interacts with the latching arrangement (18) for displacement to a desired overall length, the engagement part being insertable into the wall of the first part (1) and having a spring-prestressed ratchet part.

2. Spacer according to Claim 1, **characterized in that** an arresting part (27), which interacts with the latching arrangement (18, 19), is provided in order to arrest the two parts at the desired overall length.

3. Spacer according to Claim 2, **characterized in that** the ratchet part is designed as a spring-prestressed ball (25).

4. Spacer according to Claim 3, **characterized in that** the depressions are designed in the form of hollow ball segments.

5. Spacer according to Claim 4, **characterized in that** the distance between the centre points of two adjacent depressions is smaller than the ball diameter and is preferably smaller than the diameter of the border (20) of the depression.

6. Spacer according to one of Claims 2 to 5, **characterized in that** the arresting part is designed as a screw (25) which, on its end surface which is directed towards the latching arrangement, has a ball segment (28) which is dimensioned such that it just fits into the depression (18).

7. Spacer according to one of Claims 2 to 6, **characterized in that** the first part (1) has a first threaded bore (6) for accommodating the engagement part (23) and a second threaded bore (7), which is arranged above or beneath as seen in the direction of the centre axis (9), for accommodating the arresting part (25).

8. Spacer according to one of Claims 1 to 7, **characterized in that** the free ends of the two spacer parts are designed in the form of serrations in each case.

9. Spacer according to one of Claims 1 to 8, **characterized in that** the walls of the two spacer parts (1, 2) have a plurality of cutouts (8, 21), of which in each latching position, distributed in a circumferential direction, a plurality are at least partially congruent.

10. Spacer according to one of Claims 1 to 9, **characterized in that** the end section (5, 10) of at least one of the two spacer parts (1, 2) has rhomboidal cutouts (10, 11), and a diagonal of the rhombuses extends parallel in each case to the longitudinal axis (9) of the spacer.

11. Spacer according to one of Claims 1 to 10, **characterized in that** a stop (17) is provided in order to limit the relative displacement to a maximum overall length.

## Revendications

1. Espaceur à longueur axiale réglable destiné à être inséré entre deux corps vertébraux, comprenant une première partie (1) en forme de manchon et une deuxième partie (2) guidée dans celle-ci et pouvant coulisser dans le sens axial par rapport à la première partie pour ajuster la longueur totale, **caractérisé en ce que** la deuxième partie (2) possède sur sa paroi extérieure orientée vers la première partie (1) une portion s'étendant dans le sens axial et portant une crémaillère (18) formée d'une pluralité de renfoncements (18) voisins les uns des autres dans le sens axial et contigus les uns des autres et la première partie (1) présente un élément de prise (23) coopérant avec la crémaillère (18) en vue d'une translation jusqu'à une longueur totale souhaitée, l'élément de prise pouvant être introduit à travers la paroi de la première partie (1) et présentant une partie formant cliquet précontrainte par un ressort.

2. Espaceur selon la revendication 1, **caractérisé en ce qu'**un élément d'arrêt (27) coopérant avec la crémaillère (18, 19) est prévu pour bloquer les deux parties à la longueur totale souhaitée.

3. Espaceur selon la revendication 2, **caractérisé en ce que** la partie formant cliquet est conçue sous forme d'une bille (25) précontrainte par un ressort.

4. Espaceur selon la revendication 3, **caractérisé en ce que** les renfoncements ont la forme de segments de sphère creux.

5. Espaceur selon la revendication 4, **caractérisé en ce que** la distance entre les centres de deux renfoncements voisins est plus petite que le diamètre de la bille et de préférence plus petit que le diamètre du bord (20) du renfoncement.

6. Espaceur selon l'une ou l'ensemble des revendications 2 à 5, **caractérisé en ce que** l'élément d'arrêt est conçu sous forme d'une vis (25) qui présente sur sa face orientée vers la crémaillère un segment de sphère (28) dimensionné de telle sorte qu'il s'engage tout juste dans le renfoncement (18).

7. Espaceur selon l'une ou l'ensemble des revendications 2 à 6, **caractérisé en ce que** la première partie (1) présente un premier trou fileté (6) destiné à recevoir l'élément de prise (23) et un deuxième trou fileté (7) disposé au-dessus ou en dessous, vu dans le sens de l'axe médian (9), et destiné à recevoir l'élément d'arrêt (25).

8. Espaceur selon l'une ou l'ensemble des revendications 1 à 7, **caractérisé en ce que** les extrémités libres des deux parties de l'espaceur sont en dents de scie.

9. Espaceur selon l'une ou l'ensemble des revendications 1 à 8, **caractérisé en ce que** les parois des deux parties de l'espaceur (1, 2) présentent une pluralité d'évidements (8, 21) dont une pluralité coïncide au moins partiellement en recouvrement dans le sens de la circonférence dans chaque position de crémaillère.

10. Espaceur selon l'une ou l'ensemble des revendications 1 à 9, **caractérisé en ce que** la partie d'extrémité (5, 10) de l'une au moins des parties de l'espaceur (1, 2) présente des évidements (10, 11) en losange, une diagonale des losanges étant parallèle à l'axe longitudinal (9) de l'espaceur.

11. Espaceur selon l'une ou l'ensemble des revendications 1 à 10, **caractérisé en ce qu'**il est prévu une butée (17) destinée à limiter la translation relative à une longueur totale maximale.
